# NEUE EUROPÄISCHE PATENTSCHRIFT

(11) **EP 2 365 794 B2**
(45) Veröffentlichungstag und Bekanntmachung des Hinweises auf die Entscheidung über den Einspruch: **28.06.2017**
(45) Hinweis auf die Patenterteilung: 20.03.2013
(21) Anmeldenummer: 10704304.4
(22) Anmeldetag: 26.01.2010
(51) Int. Cl.: A61F 13/00, A61F 13/36

(54) **WUNDREINIGUNGSEINRICHTUNG**
WOUND CLEANSING ASSEMBLY
DISPOSITIF DE NETTOYAGE DE PLAIE

(30) Priorität: 28.01.2009 AT 1452009
(43) Veröffentlichungstag der Anmeldung: 21.09.2011
(73) Patentinhaber: RAU-BE Beteiligungen GmbH, 1140 Wien (AT)
(72) Erfinder: Engl Johannes, 6858 Bildstein (AT); Strohal Robert, Feldkirch-Nofels (AT)
(74) Vertreter: Seranski, Klaus
(86) Internationale Anmeldenummer: PCT/AT2010/000027
(87) Internationale Veröffentlichungsnummer: WO 2010/085831

(56) Entgegenhaltungen:
- EP-A1- 0 552 933
- WO-A1-98/46818
- DE-A1- 19 839 505
- US-A- 3 561 441
- US-A- 3 732 135
- US-A- 5 989 677
- US-A1- 2004 265 534
- US-A1- 2004 265 534
- US-A1- 2008 177 253
- US-A1- 2008 177 253

## Beschreibung

Die vorliegende Erfindung betrifft eine Wundreinigungseinrichtung nach dem Oberbegriff des Patentanspruchs 1. Weiters betrifft die Erfindung die Verwendung zur Reinigung von Wunden, eine Anordnung aus einer Wundreinigungseinrichtung und einer Verpackung sowie Verfahren zur Herstellung von solchen Wundreinigungseinrichtungen.

Der Beginn jeglicher medizinischer Wundbehandlung ist die erfolgreiche Reinigung der Wunde. Hierzu werden bisher beim Stand der Technik verschiedene Methoden und Hilfsmittel angewendet. Bekannt und weit verbreitet ist die Reinigung von Wunden mittels Baumwolltupfer. Bekannt sind auch chirurgische oder hydrochirurgische Reinigungsmethoden oder die Anwendung von Stoßwellentherapie oder Ultraschall. Die zentralen Anforderungen an eine erfolgreiche Wundreinigung sind, dass einerseits die Verschmutzung möglichst vollständig beseitigt werden soll, andererseits aber der bereits eingetretene Heilungsprozess nicht durch Zerstören bzw. Abreiben des bereits neu gebildeten intakten Wundverschlusses rückgängig gemacht werden soll. Dies gilt insbesondere bei langwierigen ärztlichen Behandlungen von chronischen Wunden.

Von besonderer Wichtigkeit bei der Behandlung von akuten Wunden und insbesondere von chronischen Wunden ist das sogenannte Debridement. Es handelt sich dabei um den Vorgang der Wundbettpräparation bei dem vom Körper selbst gebildete Substanzen oder mit anderen Worten Humanmaterial wie z.B. überschießende Flüssigkeiten, Vibrinbeläge, abgestorbenes Gewebe der Oberhaut wie z. B. überschießendes Hornmaterial oder tote Hornzellen und/oder Beläge aus abgestorbenem Gewebe (Nekrosen) entfernt werden. Zum jetzigen Zeitpunkt ist ein solches Debridement praktisch nur mit medizinisch technischen Mitteln wie der Hydrochirurgie, Stoßwellenbehandlung oder auf chirurgischem Wege zu erreichen. Weiters gibt es beim Stand der Technik die umstrittene Form der längerfristigen Anwendung speziell angefeuchteter Wundverbände, welche nach längerer Zeit des Tragens einen solchen Debridementeffekt erzielen sollen. Die beim Stand der Technik bekannten Methoden sind aufwendig und schmerzhaft, sowie zum Teil aggressiv. Das Ziel des Debridements ist es, bei der Entfernung des die Wundheilung störenden Biomaterials die jungen sprießenden neuen Hautinseln, also das Granulationsgewebe, bis zur frühen Epithelialisierung möglichst unbeeinträchtigt zu bewahren und nur die störenden Substanzen zu entfernen. Mit den beim Stand der Technik bekannten Methoden wird dieses Ziel nicht im gewünschten Maße erreicht.

Insgesamt sind die beim Stand der Technik bekannten, bis hierher geschilderten Methoden und Hilfsmittel zum Teil sehr aufwendig und kostspielig und erfüllen die genannten zentralen Anforderungen nur unzureichend.

Die EP 0 552 933 A1 offenbart einen Debridementschwamm, welcher aus mehreren Lagen eines Fasertuches aufgebaut ist. Die US 2004/0265534 A1 zeigt ein laminiertes Tuch, welches Merkmale des Oberbegriffs des Patentanspruchs 1 aufweist. In der US 2004/0265534 A1 sind verschiedenste Verwendungsmöglichkeiten, unter anderem die Verwendung im medizinischen bzw. sanitären Bereich genannt. Die US 2008/0177253 A1 offenbart eine Wundauflage. In der US-B1-3732135 sind Verfahren und Vorrichtungen zur Herstellung eines Teppichflors beschrieben. In der DE 19839505 A1 ist ein Textilvorsatz für Reinigungsgeräte und ein Waschlappen zur Reinigung menschlicher oder tierischer Haut beschrieben, bei der eine Textilstruktur aus einer Maschen- oder Webmaschenware vorgesehen ist, die sich aus der Wischfläche heraus erstreckende Mikrofasern enthaltende Schlingen, Fäden oder Fasern aufweist. In der US 3,561,441 ist eine Wundauflage nach dem Oberbegriff des Patentanspruchs 1 angegeben, bei der nicht haftende Fäden für eine ausreichende Belüftung der Wunde und Ableitung von Wundsekreten vorgesehen sind. In der US 5,989,677 ist ein Viskoseplüsch angegeben, der im tiermedizinischen Bereich zum Reinigen und Desinfizieren von Tieren eingesetzt werden soll.

Aufgabe der vorliegenden Erfindung ist es daher, eine Möglichkeit zur Wundreinigung und insbesondere zum Debridement zu schaffen, bei der eine schonende aber auch einfach durchführbare Vorgangsweise zur Wundreinigung, insbesondere zum Debridement, möglich wird, welche die Verschmutzungen zufriedenstellend beseitigt, aber den bereits eingetretenen Heilungsprozess nicht stört oder wieder rückgängig macht.

Erfindungsgemäß wird dies erreicht durch die im kennzeichnenden Teil des Patentanspruchs 1 angegebene Weiterbildung bekannter Produkte.

Es hat sich gezeigt, dass mit solchen Wundreinigungstüchern mit Fäden aus synthetischen Fasern Wunden auf sehr einfache Weise, aber mit einer hohen Effizienz und ohne bzw. mit nur minimaler Störung des bisher schon stattgefundenen Wundheilungsprozesses gereinigt werden können. Die synthetischen Fasern bewirken dabei verschiedene Vorteile. Zum Einen sind sie einfach und zuverlässig sterilisierbar. Zum Anderen binden sie durch elektrostatische Anziehung den aus der Wunde zu entfernenden Schmutz. Die aus der Wunde entfernten Schmutzpartikel werden zuverlässig zwischen den Fäden des Wundreinigungstuches gehalten und nicht mehr zurück in die Wunde abgegeben.

Weiters ist vorgesehen, dass zumindest einige, vorzugsweise zumindest 50% der, Fäden auf ihrer von der Trägerschicht abgewandten Seite frei auskragende, vorzugsweise abgeschnittene, Enden aufweisen. Günstigerweise haben zumindest 80% oder 90% der, vorzugsweise alle, Fäden auf ihrer von der Trägerschicht abgewandten Seite frei auskragende, vorzugsweise abgeschnittene, Enden. Durch diese frei auskragenden Enden entwickeln die Fäden eine Art Rasierklingeneffekt, der die Entfernung von Schmutzpartikeln und/oder störendem biologischem, insbesondere vom Körper selbst produziertem, Material aus der Wunde besonders effektiv macht. In diesem Sinne ist weiters vorgesehen, dass die Fäden schräg zu ihrer Längserstreckung verlaufende, vorzugsweise abgeschnittene, Enden bzw. Endflächen aufweisen. Unter "schräg" werden dabei alle Winkel verstanden, die weder orthogonal bzw. normal noch parallel zur Längserstreckung der Fäden verlaufen. Als Längserstreckung der Fäden wird deren Erstreckung im gestreckten Zustand verstanden. Bevorzugt schließen die schräg zu ihrer Längserstreckung verlaufenden Enden bzw. Endflächen mit der Längserstreckung des Fadens einen Winkel zwischen 70° und 80° ein. Dies bedeutet natürlich nicht, dass die Fäden beim Wundreinigungstuch immer gestreckt sein müssen. Die Fäden können zwar unterschiedlich hart bzw. borstenartig ausgebildet sein, eine gewisse Flexibilität weisen sie aber günstigerweise immer auf. Um den gewünschten Wundreinigungseffekt möglichst rasch und flächig erzielen zu können, ist erfindungsgemäß vorgesehen, dass die Fäden einen an der Trägerschicht angeordneten und von dieser abstehenden Flor bilden.

Der Begriff der Wundreinigung umfasst dabei sowohl das Entfernen von Fremdkörpern bzw. Fremdpartikeln, also nicht körpereigenen Stoffen, als auch das Debridement, also den Vorgang der Wundbettpräparation, bei dem vom Körper selbst gebildete Substanzen bzw. Humanmaterial wie überschießende Flüssigkeiten, Fibrinbeläge, abgestorbenes Gewebe der Oberhaut wie z.B. überschießendes Hornmaterial oder tote Hornzellen und/oder Beläge aus abgestorbenem Gewebe (Nekrosen) von der Haut bzw. aus der Wunde entfernt werden. Die erfindungsgemäßen Wundreinigungseinrichtungen sind für das Debridement und dort insbesondere bei der Behandlung von akuten wie auch chronischen Wunden besonders gut geeignet, da bei ihrer Verwendung das die Wundheilung störende Biomaterial besonders schonend aber auch effektiv aus der Wunde entfernt wird und gleichzeitig die jungen sprießenden neuen Hautinseln in Form des Granulationsgewebes bewahrt bleiben. Beim Debridement handelt es sich dabei um eine der wichtigsten Maßnahmen des Managements akuter wie auch chronischer Wunden, da ohne das Debridement eine Heilung nach den bekannten medizinischen Abläufen, welche zur Heilung einer Wunde führen, nicht stattfinden kann. Insbesondere beim Debridement zeichnen sich erfindungsgemäße Wundreinigungseinrichtungen dadurch aus, dass das genannte störende Biomaterial bzw. Humanmaterial durch Anwendung des Wundreinigungstuches aus der Wunde effektiv entfernt und anschließend aber zwischen den Fäden des Wundreinigungstuches gehalten und daher nicht mehr zurück in die Wunde oder auf die Haut abgegeben wird. Mit anderen Worten zeichnen sich die erfindungsgemäßen Wundreinigungseinrichtungen dadurch aus, dass sie das beim Débridement zu entfernende Material aktiv in den Bereich zwischen die von der Trägerschicht abstehenden Fäden aufnehmen und dort dann auch festhalten, sodass das bereits entfernte Biomaterial nicht versehentlich wieder in die Wunde zurückgelangt. Das Wundreinigungstuch kann in trockener wie auch in angefeuchteter Form verwendet und/oder vor der Verwendung mit in der Medizin verwendeten bzw. allgemein bekannten Flüssigkeiten getränkt werden. Bei dem Wundreinigungstuch handelt es sich um ein sterilisiertes Produkt und/oder Medizinprodukt, welches die Vorgaben der einschlägigen Normen und Gesetze für Medizinprodukte erfüllt.

Allgemein gesprochen kann mit den erfindungsgemäßen Wundreinigungseinrichtungen somit ein Verfahren zur Reinigung, insbesondere zum Debridement, einer Wunde und/oder der Haut durchgeführt werden. Bei diesem können körperfremde Verschmutzungen und/oder störendes vom Körper selbst produziertes biologisches Material bzw. Humanmaterial aus der Wunde oder von der Haut entfernt werden. Dazu muss die Wunde.lediglich mit den von der Trägerschicht abstehenden Fäden ausgewischt bzw. gereinigt werden. Besonders gut eignen sich erfindungsgemäße Wundreinigungseinrichtungen aber wie gesagt zum Debridement also einem Verfahren bei dem vom Körper selbst produziertes Biomaterial bzw. Humanmaterial aus der Wunde und/oder von der Haut entfernt wird. Mit anderen Worten kann mit den erfindungsgemäßen Wundreinigungseinrichtungen ein Verfahren zum Aufgreifen von Humanmaterial von der Haut und/oder aus einer Wunde durchgeführt werden. Insbesondere zum Debridement können erfindungsgemäße Wundreinigungseinrichtungen bei den folgend genannten Indikationen angewendet werden: So kann man z.B. gegen eine aktuelle Besiedelung der Wunde mit Bakterien in Form eines flüssigen Biofilms, welcher zur Kolonisation oder lokalen Infektion der Wunde führt, vorgehen. Es können somit erfindungsgemäße Wundreinigungseinrichtungen besonders gut dazu eingesetzt werden, bakterielle Besiedlungen der Wunde mit einem Biofilm, welcher zur systematischen Infektion bei Patienten führt, zu beseitigen. Des Weiteren können trockene Fibrinbeläge, welche zur Blockade der Wundheilung führen, indem sie den für die Heilung notwendigen Wundgrund bzw. Wundrand blockieren, entfernt werden. Das Gleiche gilt für die Reduktion von plattenartigen Nekrosen in Form von abgestorbenem Gewebe in feuchter wie auch in trockener Form. Darüber hinaus können auch Wunden und Wundränder mit überschießenden Hornproduktionen (Hyperkeratosen) und abgestorbenen Horn- bzw. Gewebszellen effektiv und schonend behandelt werden.

Die Fäden können relativ weich sein, sie können aber auch relativ steif, also borstenartig, ausgebildet sein. Die Fäden können theoretisch auch Bestandteile aus nicht synthetischen Fasern haben. Bevorzugt ist jedoch vorgesehen, dass sie ausschließlich aus synthetischen Fasern bestehen. Insgesamt bildet das Wundreinigungstuch ein textiles Flächengebilde, welches, gegebenenfalls im Anschluss an ein Anfeuchten, schnell und einfach zur, insbesondere medizinischen, Wundreinigung bzw. zum Debridement, insbesondere bei akuten oder chronischen Wunden, verwendet werden kann. Unter dem Begriff der synthetischen Faser werden zunächst allgemein alle nicht natürlichen Fasern, insbesondere Kunststofffasem, verstanden.

Von Vorteil ist auch, wenn die Trägerschicht, vorzugsweise das gesamte Wundreinigungstuch, synthetische Fasern, vorzugsweise Kunststofffasern, aufweist oder noch besser aus solchen Fasern besteht. Wundreinigungstücher dieser Art sind einfach herstellbar. Zum Beispiel kann ein Verfahren zur Herstellung eines Wundreinigungstuches vorsehen, dass zwei Trägerschichten in einem ersten Verfahrensschritt gemeinsam in Form von Maschenware hergestellt, vorzugsweise gestrickt oder gewoben werden, wobei bei diesem ersten Verfahrensschritt zwischen den beiden Trägerschichten eine Zwischenschicht aus sich zwischen den beiden Trägerschichten erstreckenden und in beide Trägerschichten eingearbeiteten Fäden ausgebildet wird und die Fäden in einem zweiten Verfahrensschritt, vorzugsweise in der Mitte zwischen den beiden Trägerschichten durchtrennt, vorzugsweise durchgeschnitten, werden. Bei einer solchen Herstellungsweise wird gleichzeitig auch erreicht, dass die Fäden schräg zu ihrer Längserstreckung verlaufende, vorzugsweise abgeschnittene Enden bzw. Endflächen aufweisen. Alternative Herstellungsverfahren sehen vor, dass ein Wundreinigungstuch, vorzugsweise durch Weben oder Stricken, mit der Trägerschicht und den von der Trägerschicht abstehenden Fäden aus synthetischen Fasern hergestellt wird und anschließend die abstehenden Fäden schräg zu ihrer Längserstreckung abgeschnitten werden. Hierdurch können die Fäden sowohl in der gewünschten Länge als auch in dem gewünschten Winkel abgeschnitten werden. Geeignete Schneideinrichtungen sind beim Stand der Technik bekannt. Bevorzugte Materialien aus denen die synthetischen Fasern der Fäden und/oder der Trägerschicht vorzugsweise des gesamten Wundreinigungstuches bestehen, sind Polyester, Polyamid und/oder Polyacryl. Diese verschiedenen Kunststoffe können sortenrein für das gesamte Wundreinigungstuch verwendet werden. Es ist aber auch möglich, die Fäden und/oder die Trägerschicht und/oder das gesamte Wundreinigungstuch aus Gemischen von synthetischen Fasern aus Polyester und/oder Polyamid und/oder Polyacryl herzustellen. Z.B. ist es möglich, für die Fäden synthetische Fasern zu 80% aus Polyacryl und zu 20% aus Polyester zu verwenden. Die Trägerschicht kann zum Beispiel zu 100% aus Polyester bestehen. Bevorzugte Ausgestaltungsformen sehen aber auch vor, die Fäden aus 100% Polyester herzustellen.

Häufig wird es vorkommen, dass das erfindungsgemäße Wundreinigungstuch als Einweg-Produkt eingesetzt und nach der Benutzung weggeworfen wird. Im Sinne einer einfachen Entsorgung ist es günstig, wenn das gesamte Wundreinigungstuch möglichst sortenrein ausgebildet ist. In diesem Sinne ist bevorzugt vorgesehen, dass die Fäden und/oder die Trägerschicht, vorzugsweise das gesamte Wundreinigungstuch, zu zumindest 90 Gew.-% (Gewichtsprozent), vorzugsweise vollständig, aus einem einzigen synthetischen Material bzw. Kunststoff, vorzugsweise aus Polyester oder Polyamid oder Polyacryl, besteht. In allen Ausgestaltungsformen können die Fäden und/oder die Trägerschicht mit einer Beschichtungsmasse, vorzugsweise aus 100% Polyacryl, beschichtet sein. Die Beschichtungsmasse kann mit Hilfe eines Zylinders flüssig auf die Fasern aufgestrichen und eingewalzt werden. Günstig ist es, zwischen 50 und 70 Gramm Beschichtungsmasse pro Quadratmeter zu verwenden, bevorzugt sind dabei 60 g/m2.

Es kann im Rahmen der Erfindung aber auch vorgesehen sein, dass zumindest ein Teil der von der Trägerschicht abstehenden Fäden und/oder zumindest ein Teil der die Trägerschicht bildenden Fäden, vorzugsweise reine, Silberfäden und/oder, vorzugsweise reine, Kupferfäden sind und/oder synthetische Fasern mit einer Silberbeschichtung und/oder Kupferbeschichtung enthalten oder daraus gebildet sind. Durch das Silber bzw. Kupfer kann eine vorübergehende oder permanente antibakterielle Wirkung erzielt werden. Es kann sich um reine Silber- bzw. Kupferfäden, also um Fäden, die im Rahmen des bei Herstellung erreichbaren Reinheitsgrades ausschließlich aus Silber oder Kupfer bestehen, oder um Fäden, die Silber oder Kupfer enthalten, handeln. Eine Beschichtung von synthetischen Fasern mit Silber bzw. Kupfer kann durch Einlegen der Fasern oder der fertig hergestellten Wundreinigungstücher in Silber- bzw. Kupferbäder oder durch entsprechendes Besprühen oder Bedampfen erreicht werden. Die Fäden können in all diesen Fällen in der Trägerschicht und/oder auch im von den Fäden gebildeten Flor enthalten sein.

Zur Erreichung der gewünschten vorübergehenden oder aber auch permanenten antibakteriellen Wirkung kann auch vorgesehen sein, dass an synthetischen Fasern der Trägerschicht und/oder an den synthetischen Fasern der von der Trägerschicht abstehenden Fäden Nanopartikel anhaften bzw. angeordnet sind. Hierzu können die Fasern oder aber auch das Wundreinigungstuch bzw. die Wundreinigungseinrichtung in Bäder mit entsprechenden Nanopartikeln eingelegt oder mit Nanopartikeln besprüht oder bedampft werden. Die Nanopartikel werden vom Faserkern aufgenommen, was zur gewünschten antibakteriellen Wirkung führt. Mit Hilfe der Nanoteilchen könnten aber auch weitere zusätzliche Eigenschaften erreicht werden, wie z.B. eine Desinfektion.

Die Verwendung von, vorzugsweise ausschließlich, synthetischen Fasern, vorzugsweise Kunststofffasern, hat des Weiteren den Vorteil, dass nachgewiesener Maßen durch solche Fasern keine allergischen Reaktionen hervorgerufen werden.

Wie bereits dargelegt, bezieht sich die Erfindung somit insbesondere auf ein Tuch zur Verwendung bzw. zur spezifischen Anwendung als Wundreinigungseinrichtung bzw. als Wundreinigungstuch. Die Erfindung bezieht sich somit auch auf ein Tuch zur Reinigung, insbesondere zum Debridement, von Wunden oder der Haut, welches eine Trägerschicht und an der Trägerschicht angeordnete und von der Trägerschicht abstehende Fäden, vorzugsweise ausschließlich, aus synthetischen Fasern, vorzugsweise Kunststofffasern, aufweist.

Die bevorzugten Ausgestaltungsformen dieses Tuches bzw. Wundreinigungstuches bzw. der Wundreinigungseinrichtung sind bereits zum Teil erläutert worden und werden anhand der, in der Figurenbeschreibung dargelegten Ausführungsbeispiele noch einmal vertieft erläutert. In diesem Zug ist festzustellen, dass sich die Erfindung somit auch auf eine Verwendung von synthetischen Fasern zur Herstellung einer Wundreinigungseinrichtung oder eines Wundreinigungstuches einer Wundreinigungseinrichtung mit den genannten Eigenschaften zur Reinigung bzw. zum Debridement von Wunden oder der Haut bezieht. Bei den genannten Verwendungen bzw. spezifischen Anwendungen steht die medizinische Anwendung durch den Arzt, also insbesondere die chirurgische oder therapeutische Behandlung, von, insbesondere akuten oder chronischen, Wunden im Vordergrund.

Um zur Wundreinigung bzw. zum Debridement ein steriles Wundreinigungstuch bzw. Tuch zur Verfügung stellen zu können, sieht die Erfindung darüber hinaus eine Anordnung vor, welche zumindest ein Wundreinigungstuch oder zumindest ein entsprechendes Tuch und eine vorzugsweise luftdicht verschlossene Packung, vorzugsweise Kunststoffverpackung aufweist, wobei das zumindest eine Wundreinigungstuch oder Tuch steril in der Verpackung verpackt ist.

Weitere Merkmale und Einzelheiten verschiedener Ausführungsvarianten der vorliegenden Erfindung ergeben sich aus der nachfolgenden Figurenbeschreibung.

Dabei zeigt:
Fig. 1 eine schematisierte Seitenansicht eines erfindungsgemäßen Wundreinigungstuches;
Fig. 2 einen gerade abgeschnittenen Faden gemäß Stand der Technik;
Fig. 3 einen erfindungsgemäßen Faden mit schräg verlaufenden Endflächen;
Fig. 4 ein erfindungsgemäßes Ausführungsbeispiel in Form eines handschuhartigen Wundreinigungstuches;
Fig. 5 eine schematisierte Skizze zur Erläuterung eines bevorzugten Herstellungsverfahrens;
Fig. 6 eine erfindungsgemäße Anordnung mit einem in einer Verpackung steril verpackten Wundreinigungstuch;
Fig. 7 eine erfindungsgemäße Wundreinigungseinrichtung mit einer Verstärkungsschicht und einer Schlaufe zur besseren Handhabung;
Fig. 8 ein weiteres erfindungsgemäßes Ausführungsbeispiel einer Wundreinigungseinrichtung mit einer Verstärkungsschicht und einer zum Hineingreifen vorgesehenen Tasche;
Fig. 9 ein weiteres erfindungsgemäßes Ausführungsbeispiel bei dem das Wundreinigungstuch an einem Tragkörper befestigt ist und
Fig. 10 eine weitere erfindungsgemäße Wundreinigungseinrichtung bei dem das Wundreinigungstuch ebenfalls an einem Tragkörper befestigt ist.

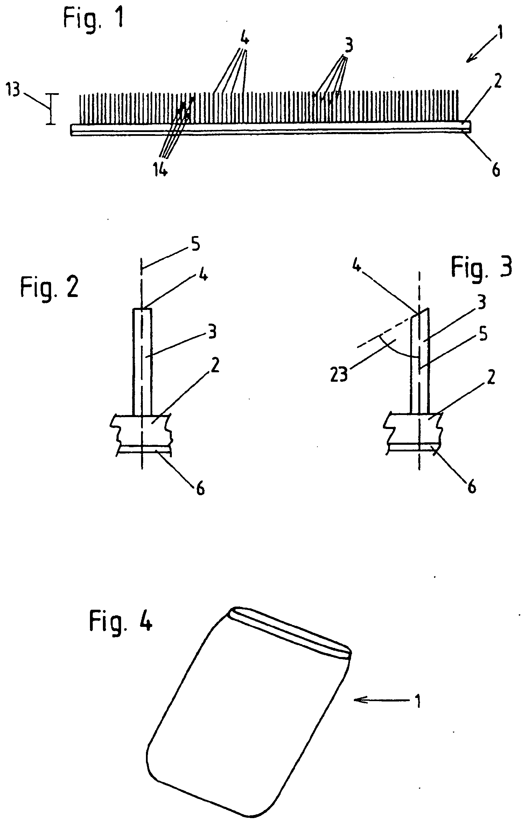

Wie in der Seitenansicht gemäß Fig. 1 zu sehen, sind die Fäden 3 an der Trägerschicht 2 befestigt. Sie stehen seitlich von der Trägerschicht 2 ab und sind günstigerweise so dicht nebeneinander angeordnet, dass sie.eine Art Flor bilden. Auch wenn die Fäden in der gezeigten schematischen Darstellung linear verlaufend dargestellt sind, so ist es letztendlich eine Frage ihrer Steifigkeit bzw. Biegbarkeit, inwiefern die Fäden 3 tatsächlich, wie hier dargestellt, borstenartig von der Trägerschicht 2 abstehen. Selbst im unbelasteten, hier dargestellten Zustand muss nicht zwingend vorgesehen sein, dass alle Fasern 3 linear längserstreckt von der Trägerschicht 2 abstehen. Ihre Biegbarkeit sollte auf jeden Fall so ausgestaltet sein, dass sie, sobald das Wundreinigungstuch gegen die Wunde gedrückt wird, sich entsprechend verbiegen. Die Härte bzw. Biegbarkeit der Fäden kann durch ihre Dicke und das verwendete Material wunschgemäß eingestellt werden. Verwendete Fäden 3 weisen zwischen 0,5 und 20 D-Tex auf (1D-Tex = 1g/10000m). Besonders bevorzugte Ausgestaltungsformen sehen vor, dass die Fäden 6,7 D-Tex aufweisen. Die Florhöhe 13 bzw. Länge der Fäden 3 beträgt zwischen 3mm und 30mm, vorzugsweise zwischen 3 und 12 mm, besonders bevorzugt ist eine Florhöhe 13 von 8mm. Günstigerweise werden für die Trägerschicht 2 und/oder die Fäden 3 Fasern mit einem Flächengewicht zwischen 500 und 900 g/m2 (Gramm pro Quadratmeter) bzw. besonders bevorzugt von ca. 700 g/m2 eingesetzt. Die Trägerschicht 2 ist günstigerweise in Form einer Maschenware ausgebildet. Es kann sich z.B. um ein Gewebe oder um Strickware handeln. Die von der Trägerschicht 2 abstehenden Fäden 3 sind günstigerweise in die Trägerschicht 2 eingewoben bzw. eingestrickt. Besonders bevorzugt handelt es sich bei der Maschenware um eine Gewebe. Bei diesem halten die Fasern bzw. die Fäden 3 besonders gut an der Trägerschicht 2, sodass sie sich nicht beim Vorgang der Wundreinigung bzw. des Debridements versehentlich von der Trägerschicht 2 lösen können. Darüber hinaus tritt bei Geweben auch kein Fuseln also kein Loslösen von Einzelfasern der Fäden 3 auf. Insbesondere die Gewebe können aufgrund ihrer hohen Eigenfestigkeit mit oder ohne Verstärkungsschicht 6 ausgebildet sein. Die gegebenenfalls vorhandenen Silber- bzw. Kupferfäden können mit eingewoben werden. Günstigerweise weist die Trägerschicht 2 zwischen 7 und 12, bevorzugt 9, Maschen/cm2 und zwischen 10 bis 14, bevorzugt 12, Reihen/cm2 auf. Um die als Maschenware ausgebildete Trägerschicht 2 zu stabilisieren, kann in bevorzugten Ausgestaltungsformen, wie z.B. in Fig. 1 gezeigt, die Trägerschicht 2, vorzugsweise auf der von den abstehenden Fäden 3 abgewandten Seite, mit einer die Maschen der Trägerschicht 2 verbindenden, vorzugsweise durchgehenden, Verstärkungsschicht beschichtet sein. Die Verstärkungsschicht 6 kann aber nicht nur bei als Gewebe ausgeführten Trägerschichten 2 eingesetzt werden. Es kann vielmehr allgemein vorgesehen sein, dass in und/ oder an der Trägerschicht 2, vorzugsweise auf der von den abstehenden Fäden 3 abgewandten Seite der Trägerschicht 2 zumindest eine Verstärkungsschicht 6 angeordnet ist. Es können eine oder mehrere an der Trägerschicht 2 angeordnete Verstärkungsschichten 6 vorgesehen sein. Mittels der Verstärkungsschicht bzw. -schichten 6 wird jedenfalls einerseits erreicht, dass die Fäden 3 besonders fest an der Trägerschicht 2 halten. Zum Anderen wird aber auch die Steifigkeit der Trägerschicht 2 erhöht, was dazu führt, dass bei der Behandlung der Wunde oder der Haut der auf das Wundreinigungstuch ausgeübte Druck in der Fläche gleichmäßiger verteilt auf die Wunde einwirkt. Die Verstärkungsschicht (en) 6 ist (sind) in der Regel als zusätzliche Schicht(en) ausgebildet, welche durch geeignete Maßnahmen an der Trägerschicht befestigt ist (sind). Diese Art der Verstärkung mittels der zumindest einen Verstärkungsschicht 6 kann auf verschiedene Arten und Weisen erreicht werden. Z.B. kann die Verstärkungsschicht 6 in Form einer aushärtenden Klebschicht auf die von den Fäden 3 abgewandte Rückseite der Trägerschicht 2 aufgebracht werden. Bei der Verstärkungsschicht 6 kann es sich aber auch um eine Beschichtung aus synthetischen Stoffen, die unter Wärme flüssig werden und beim Abkühlen aushärten, handeln. Als Material kann hier z.B. Polyester zum Einsatz kommen. Die Flächengewichte solcher Beschichtungen liegen günstigerweise zwischen 40g und 120g pro m2. Alternativ kann eine Verstärkungsschicht 6 aber auch als Membrane aus synthetischen Stoffen gefertigt sein. Auch hier können vorzugsweise Polyestermaterialien verwendet werden. Es handelt sich bei den Membranen um dünne Häutchen bzw. elastische Filme. Insbesondere diese können als Trennschichten dienen. Die Membranen können bezüglich Feuchtigkeit bzw. Flüssigkeiten undurchlässig oder teilweise durchlässig aber auch vollständig durchlässig sein. Die Membrane können z.B. als elastische Filme durch Punktlaminierung mit speziellen Klebstoffen auf die Trägerschicht 2 aufgetragen werden. Alternativ ist es aber auch möglich, die Membrane durch Spritzlaminierung mit Klebstoff in Form eines elastischen Filmes auf die Trägerschicht 2 aufzubringen. Sinnvolle Flächengewichte bei den Membranen liegen zwischen 10g und 60g pro m2. Alternativ kann die Verstärkungsschicht 6 aber auch in Form einer Gummierung aus synthetischen Stoffen wie z.B. Polyestermaterialien oder natürlichen Stoffen wie z.B. Kautschuk ausgeführt werden. Die Laminierung ist mit allen gängigen Systemen möglich. Das Flächengewicht solcher Verstärkungsschichten 6 in Form von Gummierung liegt günstigerweise zwischen 20 und 60 g pro m2. Eine weitere Variante einer Verstärkungsschicht 6 sieht vor, dass diese aus einem Schaumstoff aus synthetischem Material wie z.B. Polyester besteht. Das Flächengewicht geeigneter Schaumstoffe liegt vorzugsweise zwischen 10 und 60 g pro m2. Die Befestigung an der Trägerschicht 2 kann wiederum über Laminierung erfolgen. Die Dicke solcher Verstärkungsschichten 6 aus Schaumstoff kann z.B. zwischen 5 und 20mm betragen. Insbesondere wenn manSchaumstoff als eine Verstärkungsschicht 6 vorsieht, können noch weitere Verstärkungsschichten 6 wie z.B. Membrane oder Gummierungen an der Trägerschicht 2 vorgesehen sein, um eine Feuchtigkeits- bzw. Flüssigkeitsundurchlässigkeit zu erreichen.

Um ein weiteres Beispiel zu nennen, kann eine der oder die Verstärkungsschicht 6 auch als gestricktes bzw. gewebtes Fasergeflecht aus einem synthetischen Material gebildet sein. Dieses Fasergeflecht kann z.B. auf die Trägerschicht 2 aufgeklebt werden. Das Geflecht kann wie jede andere Verstärkungsschicht 6 oder die Trägerschicht 2 selbst auch mit Noppen, z.B. aus Gummi, versehen werden, um einen besseren Halt zu gewährleisten. Allgemein ist darauf hinzuweisen, dass je nach gewünschten Eigenschaften eine oder verschiedene Verstärkungsschichten 6 an der Trägerschicht 2 angeordnet sein kann. Die Verstärkungsschichten 6 können flüssigkeits- bzw. feuchtigkeitsabweisend oder - undurchlässig ausgeführt sein, damit die die Behandlung durchführende Person nicht mit den aus den Wunden bzw. von der Haut entfernten Stoffen in Berührung kommt. Ein anderer wichtiger Effekt, welcher durch die Verstärkungsschicht(en) 6 erreicht werden kann, ist die Aussteifung des Wundreinigungstuches (1) bzw. der Wundreinigungseinrichtung.

Fig. 2 zeigt nun in einem Detail eine Ausgestaltungsform gemäß Stand der Technik, bei der die frei auskragenden Enden 4 der Fäden 3 orthogonal bzw. normal zur Längserstreckung 5 der gestreckt angeordneten Fäden 3 angeordnet sind. Fig. 3 zeigt eine erfindungsgemäße Ausgestaltungsform, bei der die frei auskragenden Enden 4, bzw. deren Endflächen nicht orthogonal sondern schräg zur Längserstreckung der Fäden 3 verlaufen. Hierdurch wird ein besonders guter rasierklingenartiger Effekt erzeugt, der die Schmutzpartikel bzw. das biologische Material besonders effektiv aus der Wunde entfernt. Der Winkel 23 zwischen den frei auskragenden Enden 4 bzw. deren Endflächen und der Längserstreckung 5 der Fäden 3 liegt günstigerweise zwischen 70° und 80°. Zurückkommend auf Fig. 1 wird noch auf eine weitere positive Eigenschaft der Verwendung von synthetischen Fasern für die Fäden 3 hingewiesen. Wie schematisiert angedeutet, bleiben die einmal aus der Wunde herausgelösten Schmutzpartikel 14 bzw. das herausgelöste biologische Material einerseits durch die Dichte des durch die Fäden 3 gebildeten Flores, andererseits aber auch durch die elektrostatische Anziehung der synthetischen Fasern der Fäden 3 im Flor gefangen, sodass keine Gefahr besteht, dass sie bei weiterer Verwendung des Wundreinigungstuches 1 wieder in die Wunde zurückkehren können.

Bei bevorzugten Ausgestaltungsformen der Erfindung kann vorgesehen sein, dass synthetische Fasern der Trägerschicht 2 und/oder die synthetischen Fasern der von der Trägerschicht 2 abstehenden Fäden 3 hitzebehandelt, vorzugsweise mittels Hitzebehandlung, geschrumpft sind. Das Erhitzen kann z.B. bei 100-200° erfolgen. Durch den Erhitzungsvorgang werden die Fasern und damit die Fäden 3 stabiler und gegebenenfalls farbechter. Durch die erhöhte Stabilität der Fäden 3 wird die Wirkung der Wundreinigungseinrichtung verbessert. Das Erhitzen kann an der Rohfaser vor Herstellung des Wundreinigungstuches 1 aber auch am ansonsten bereits fertig hergestellten Wundreinigungstuch 1 durchgeführt werden. Durch das Erhitzen kann es zu einer Schrumpfung kommen, welche z.B. zwischen 10 und 20% der vorherigen Länge der Fäden 3 bzw. Faser liegen kann.

Fig. 4 zeigt ein handschuhartig ausgebildetes, erfindungsgemäßes Ausführungsbeispiel eines Wundreinigungstuches. Dies kann z.B. durch entsprechendes Vernähen von zwei in Fig. 1 dargestellten Wundreinigungstüchern hergestellt werden, wobei die Fäden 3 nach außen weisen.

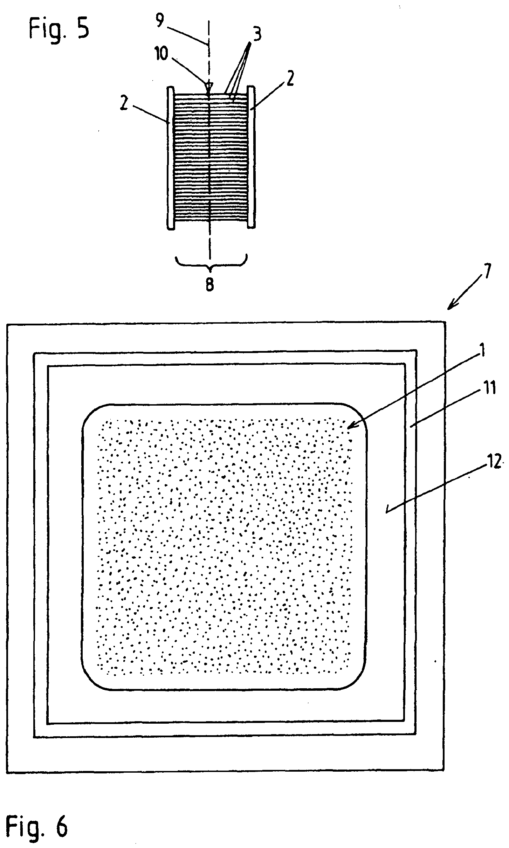

Fig. 5 zeigt eine schematisierte Skizze anhand derer ein bevorzugtes Herstellungsverfahren für erfindungsgemäße Wundreinigungstücher 1 bzw. Tücher erläutert wird. Dieses Verfahren sieht vor, dass zwei Trägerschichten 2 in einem ersten Verfahrensschritt gemeinsam in Form von Maschenware hergestellt werden, wobei bei diesem ersten Verfahrensschritt zwischen den beiden Trägerschichten 2 eine Zwischenschicht 8 aus sich zwischen den beiden Trägerschichten erstreckenden und in beide Trägerschichten 2 eingearbeiteten Fäden ausgebildet wird. Es können hierbei z.B. an sich bekannte Strick- oder Webtechniken angewendet werden. Die Fäden 3 werden günstigerweise gleich beim Stricken bzw. Weben der Trägerschichten 2 mit eingestrickt bzw. eingewoben. Solche Strick- bzw. Webtechniken sind beim Stand der Technik an sich bekannt und müssen nicht weiter erläutert werden. Ist diese Art der Maschenware, wie sie in Fig. 5 schematisiert gezeigt ist, fertiggestellt, so werden die Fäden 3 in einem zweiten Verfahrensschritt, vorzugsweise in der Mitte zwischen den beiden Trägerschichten 2 durchtrennt, vorzugsweise durchgeschnitten. In Fig. 5 sind schematisiert das Trennwerkzeug bzw. Messer 10 und die Trennlinie 9 dargestellt. Wendet man auf das Trennwerkzeug bzw. Messer 10 einen gewissen Druck beim Durchschneiden bzw. Durchtrennen der Fäden 3 an, so werden hierdurch automatisch die bevorzugten, in Fig. 3 schematisiert gezeigten, schräg zur Längserstreckung 5 der Fäden 3 angeordneten, frei auskragenden Enden 4 der Fäden 3 hergestellt.

Anschließend an die bereits geschilderten Verfahrensschritte kann, soweit gewünscht, auf den von den Fäden 3 abgewandten Rückseiten der Trägerschichten 2 noch jeweils die bereits beschriebene Verstärkungsschicht 6 angebracht werden. Darüber hinaus ist es auch möglich, auch die eingangs bereits erwähnte Beschichtungsmasse, vorzugsweise aus 100% Polyacryl, flüssig auf die Fasern, vor allem der Fäden 3, aufzustreichen bzw. in diese einzuwalzen.

Alternativ kann auch ein Herstellungsverfahren vorgesehen sein, bei dem ein Wundreinigungstuch 1, vorzugsweise durch Weben oder Stricken, mit der Trägerschicht 2 und den von der Trägerschicht 2 abstehenden Fäden 3 aus synthetischen Fasern separat hergestellt wird und anschließend die abstehenden Fäden 3 schräg zu ihrer Längserstreckung 5 abgeschnitten werden. Fig. 1 zeigt ein solches Wundreinigungstuch 1 nach dem Abschneiden der Fäden 3. Die Fäden können sowohl in der gewünschten Länge als auch in dem gewünschten Winkel abgeschnitten werden. Geeignete Schneideinrichtungen sind beim Stand der Technik bekannt. Eine oder mehrere gegebenenfalls vorgesehene Verstärkungsschichten 6 können vor oder aber auch nach dem Abschneiden der Fäden 3 angebracht werden.

In Fig. 6 ist darüber hinaus noch eine erfindungsgemäße Anordnung aus einer Verpackung 7 und einem erfindungsgemäßen Wundreinigungstuch 1 der geschilderten Art gezeigt. Das Wundreinigungstuch 1 ist steril in der Verpackung 7 verpackt. Die Verpackung 7 kann z.B. als Kunststoffverpackung bzw. Folienverpackung ausgeführt sein. Wie in Fig. 6 angedeutet, kann es sich z.B. um einen zweischichtigen Aufbau handeln. Dieser kann aus einer Deckfolie 12 und einem auf der anderen Seite angeordneten Sterilisationspapier bestehen, wobei Deckfolie 12 und Sterilisationspapier mittels einer umlaufend geschlossenen Schweißnaht 11 miteinander verschweißt sein können. Es ist aber auch die Verwendung von anderen, beim Stand der Technik an sich bekannten, sterilisierten Verpackung möglich. Das Wundreinigungstuch 1 kann vor der Verpackung durch an sich bekannte Sterilisationsverfahren wie Dampfsterilisation, Gammastrahlensterilisation oder Sauerstoffsterilisation sterilisiert werden. Die Kantenlänge des Wundreinigungstuches 1 bzw. auch der Verpackung liegt günstigerweise im Bereich zwischen 5 und 20cm, vorzugsweise zwischen 10 und 20cm.

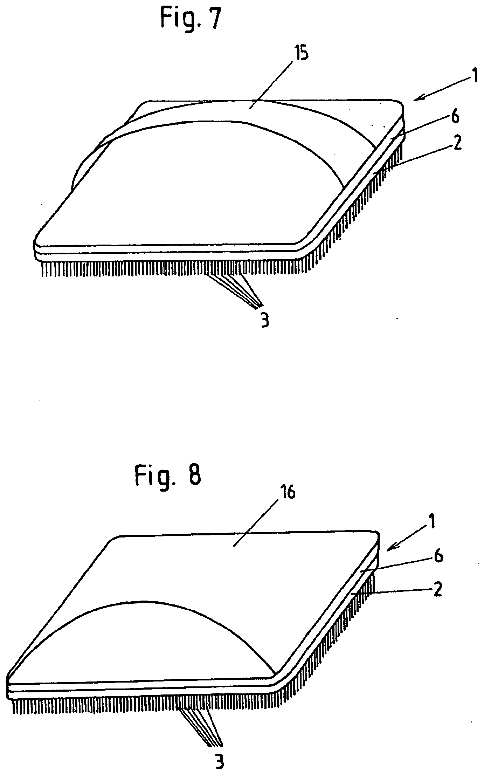

In Fig. 7 wird ein weiteres erfindungsgemäßes Ausführungsbeispiel einer Wundreinigungseinrichtung gezeigt. Auf der Trägerschicht 2 ist auf der von den Fäden 3 abgewandten Seite eine Verstärkungsschicht 6 aufgebracht. Diese kann bei der Wahl entsprechender Materialien und Dicken zum Einen für eine höhere Steifigkeit des Wundreinigungstuches sorgen. Zum Anderen kann die Verstärkungsschicht 6 bei einer feuchtigkeits- bzw. flüssigkeitsundurchlässigen Ausführung auch die Hand der die Wundbehandlung durchführenden Person vor einem in Kontakt kommen mit dem aus der Wunde entfernten Material schützen. Um die Handhabung zu vereinfachen, ist im Ausführungsbeispiel gemäß Fig. 7 eine Schlaufe 15 am Rücken bzw. an der von den Fäden 3 abgewandten Seite der Trägerschicht 2 angebracht. In diese Schlaufe 15 kann die die Behandlung durchführende Person mit der Hand hineingreifen, sodass die Wundreinigungseinrichtung beim Auswischen bzw. Reinigen der Wunde mittels der Fäden 3 sicher und fest gehalten werden kann. Die Schlaufe 15 kann aus elastischem aber auch unelastischem Material bestehen. Das Gleiche gilt für das Ausführungsbeispiel gemäß Fig. 8, bei dem anstelle der Schlaufe 15 eine Tasche 16 vorgesehen ist, in die, die Behandlung ausführende Person mit der Hand hineingreifen kann. Wie auch bei allen anderen Ausführungsbeispielen kann die von den Fäden 3 abgewandte Seite des Wundreinigungstuches 1, an der die die Behandlung durchführende Person anfasst, mit Noppen versehen sein. Dies erhöht die Rutschfestigkeit.

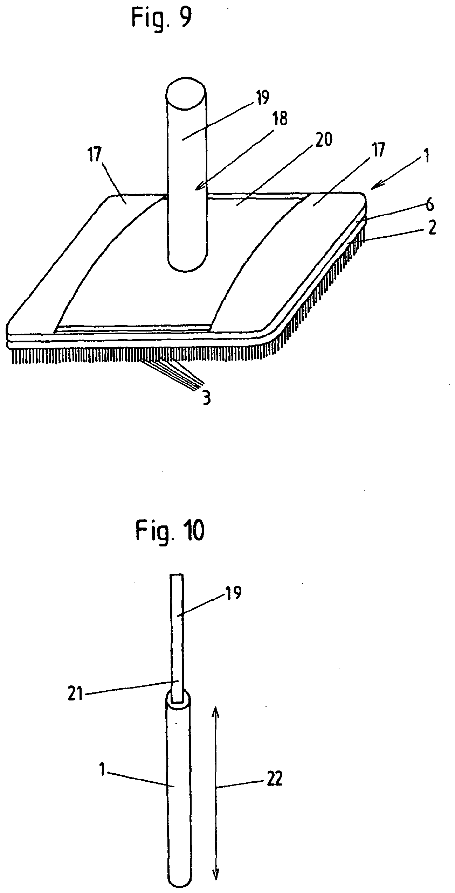

Bei anderen wie z.B. in den Fig. 9 und 10 gezeigten Ausgestaltungsformen der Erfindung kann auch vorgesehen sein, dass das Wundreinigungstuch 1 an einem, vorzugsweise starren, Trägerkörper, vorzugsweise austauschbar, angeordnet und/oder befestigt ist. Die Verwendung solcher Tragkörper 18 bietet sich insbesondere an, wenn das Wundreinigungstuch 1 für die Behandlung kleinerer oder tieferer Wunden kleinere Außenabmessungen haben soll. So kann z.B. vorgesehen sein, dass das Wundreinigungstuch 1 Kantenlängen zwischen 3 cm und 6 cm, vorzugsweise von 5 cm, aufweist. Der Tragkörper 18 wird im gezeigten Ausführungsbeispiel aus einer Platte 20 und einem daran angeordneten Griff 19 gebildet. Beides ist günstigerweise wie der gesamte Tragkörper 18 im Wesentlichen starr ausgebildet. In der gezeigten Variante erfolgt die Befestigung des Tragkörpers 18 bzw. eine Platte 20 über das Einführen der Platte 20 in Einstecktaschen bzw. Bänder 17 am Rande des Wundreinigungstuches 1. Natürlich sind auch andere Befestigungsvarianten wie z.B. Klettverschlüsse, an denen gegebenenfalls auch andere Gegenstände befestigt werden können, denkbar und möglich.

In Fig. 10 ist eine erfindungsgemäße Variante einer Wundreinigungseinrichtung gezeigt, bei der das Wundreinigungstuch 1 an einem Stiel 21 befestigt ist. Der Stiel 21 weist wiederum einen Griff 19 auf, an dem die Wundreinigungseinrichtung angefasst werden kann. Die nach außen abstehenden Fäden 3 des Wundreinigungstuches 1 sind in Fig. 10 nicht extra dargestellt. In den Varianten gemäß Fig. 9 und 10 wie auch in anderen solchen Ausgestaltungsformen kann das Wundreinigungstuch 1 lösbar oder fix am Tragkörper 18 befestigt sein. Erfindungsgemäße Ausgestaltungsformen mit vorzugsweise im Wesentlichen steifen Tragkörpern 18 sind besonders dann gut geeignet, wenn es darum geht, gezielten Druck auf die gewünschte zu behandelnde Stelle bzw. Wunde auszuüben. Die Variante gemäß Fig. 10 bietet sich besonders dann an, wenn es darum geht, kleine und schwer zugängliche Bereiche einer Wunde oder solche Hautbereiche zu behandeln. Der Stiel 21 ist günstigerweise innerhalb des Wundreinigungstuches 1 weitergeführt um dieses entsprechend auszusteifen. Das Wundreinigungstuch 1 kann hierzu z.B. um den Stiel 21 herumgewickelt werden bzw. eine entsprechende Tasche bilden, in die der Stiel 21 eingeführt wird.

Die Vielzahl der gezeigten Ausgestaltungsformen der Erfindung soll darlegen, dass die Erfindung nicht auf die konkret dargestellten Varianten beschränkt ist. Vielmehr ist es möglich, die genannten bevorzugten Ausgestaltungsformen und Merkmale entsprechend zu kombinieren, um weitere, für den jeweiligen Einsatz besonders günstige Varianten von erfindungsgemäßen Wundreinigungseinrichtungen bereitzustellen.

### Legende zu den Hinweisziffern:

1 Wundreinigungstuch
2 Trägerschicht
3 Fäden
4 Ende
5 Längserstreckung
6 Verstärkungsschicht
7 Verpackung
8 Zwischenschicht
9 Trennlinie
10 Trennwerkzeug
11 Schweißnaht
12 Deckfolie
13 Florhöhe
14 Schmutzpartikel
15 Schlaufe
16 Tasche
17 Einstecktasche oder -band
18 Tragkörper
19 Griff
20 Platte
21 Stiel
22 Länge
23 Winkel

## Patentansprüche

1. Wundreinigungseinrichtung, welche ein Wundreinigungstuch (1) aufweist oder ist, welches zumindest eine Trägerschicht (2) und an der Trägerschicht (2) angeordnete und von der Trägerschicht (2) abstehende Fäden (3) aus synthetischen Fasern aufweist, wobei zumindest einige der Fäden (3) auf ihrer von der Trägerschicht (2) abgewandten Seite frei auskragende, vorzugsweise abgeschnittene, Enden (4) aufweisen, wobei die Fäden (3) schräg zu ihrer Längserstreckung (5) verlaufende, vorzugsweise abgeschnittene, Enden (4) bzw. Endflächen aufweisen und die von der Trägerschicht abstehenden Fäden einen an der Trägerschicht angeordneten und von dieser abstehenden Flor bilden, und das Wundreinigungstuch steril in einer vorzugsweise luftdicht verschlossenen Verpackung (7) verpackt ist, **dadurch gekennzeichnet, dass** die Florhöhe zwischen 3 und 30 mm beträgt und die von der Trägerschicht abstehenden Fäden zwischen 0,5 und 20 dtex aufweisen.

2. Wundreinigungseinrichtung nach Anspruch 1, **dadurch gekennzeichnet, dass** die Trägerschicht(2), vorzugsweise das gesamte Wundreinigungstuch (1), synthetische Fasern, vorzugsweise Kunststofffasern, aufweist, vorzugsweise aus synthetischen Fasern, vorzugsweise Kunststofffasern, besteht und/oder dass vorgesehen ist, dass die synthetischen Fasern der Fäden (3) und/oder der Trägerschicht (2), vorzugsweise des gesamten Wundreinigungstuches (1), aus Polyester und/oder Polyamid und/oder Polyacryl bestehen.

3. Wundreinigungseinrichtung nach Anspruch 1 oder 2, **dadurch gekennzeichnet, dass** die Fäden (3) und/oder die Trägerschicht (2) mit einer Beschichtungsmasse, vorzugsweise aus 100% Polyacryl, beschichtet sind (ist).

4. Wundreinigungstuch nach einem der Ansprüche 1 bis 3, **dadurch gekennzeichnet, dass** die Trägerschicht (2) Maschenware, vorzugsweise ein Gewebe oder Strickware, ist und vorzugsweise die von der Trägerschicht (2) abstehenden Fäden (3) in die Trägerschicht (2) eingewoben oder eingestrickt sind.

5. Wundreinigungseinrichtung nach Anspruch 4, **dadurch gekennzeichnet, dass** die Trägerschicht (2), vorzugsweise auf der von den abstehenden Fäden (3) abgewandten Seite, mit einer die Maschen der Trägerschicht (2) verbindenden, vorzugsweise durchgehenden, Verstärkungsschicht (6) beschichtet ist.

6. Wundreinigungseinrichtung nach einem der Ansprüche 1 bis 5, **dadurch gekennzeichnet, dass** zumindest ein Teil der von der Trägerschicht (2) abstehenden Fäden (3) und/oder zumindest ein Teil der die Trägerschicht (2) bildenden Fäden (3), vorzugsweise reine, Silberfäden und/oder, vorzugsweise reine, Kupferfäden sind und/oder synthetische Fasern mit einer Silberbeschichtung und/oder Kupferbeschichtung enthalten oder daraus gebildet sind.

7. Wundreinigungseinrichtung nach einem der Ansprüche 1 bis 6, **dadurch gekennzeichnet, dass** an synthetischen Fasern der Trägerschicht (2) und/oder an den synthetischen Fasern der von der Trägerschicht (2) abstehenden Fäden (3) Nanopartikel anhaften bzw. angeordnet sind.

8. Wundreinigungseinrichtung nach einem der Ansprüche 1 bis 7, **dadurch gekennzeichnet, dass** das Wundreinigungstuch (1) an einem, vorzugsweise starren, Trägerkörper (18) der Wundreinigungseinrichtung, vorzugsweise austauschbar, angeordnet und/oder befestigt ist.

9. Wundreinigungseinrichtung nach einem der Ansprüche 1 bis 8, **dadurch gekennzeichnet, dass** in und/ oder an der Trägerschicht (2), vorzugsweise auf der von den abstehenden Fäden (3) abgewandten Seite der Trägerschicht (2), zumindest eine Verstärkungsschicht (6) angeordnet ist.

10. Wundreinigungseinrichtung nach einem der Ansprüche 1 bis 9, **dadurch gekennzeichnet, dass** synthetische Fasern der Trägerschicht (2) und/oder die synthetischen Fasern der von der Trägerschicht (2) abstehenden Fäden (3) hitzebehandeft, vorzugsweise mittels Hitzebehandlung geschrumpft, sind.

11. Verwendung von synthetischen Fasern zur Herstellung einer Wundreinigungseinrichtung oder eines Wundreinigungstuches (1) einer Wundreinigungseinrichtung nach einem der Ansprüche 1 bis 10 zur Reinigung, vorzugsweise zum Debridement, von Wunden oder der Haut.

12. Verfahren zur Herstellung einer Wundreinigungseinrichtung nach einem der Ansprüche 1 bis 10, **dadurch gekennzeichnet, dass** zwei Trägerschichten (2) in einem ersten Verfahrensschritt gemeinsam in Form von Maschenware hergestellt, vorzugsweise gestrickt oder gewoben, werden, wobei bei diesem ersten Verfahrensschritt zwischen den beiden Trägerschichten (2) eine Zwischenschicht (8) aus sich zwischen den beiden Trägerschichten (2) erstreckenden und in beide Trägerschichten (2) eingearbeiteten Fäden (3) ausgebildet wird und die Fäden (3) in einem zweiten Verfahrensschritt, vorzugsweise in der Mitte zwischen den beiden Trägerschichten (2), durchtrennt, vorzugsweise durchgeschnitten, werden.

13. Verfahren zur Herstellung einer Wundreinigungseinrichtung oder eines Tuches nach einem der Ansprüche 1 bis 10, **dadurch gekennzeichnet, dass** ein Wundreinigungstuch, vorzugsweise durch Weben oder Stricken, mit der Trägerschicht und den von der Trägerschicht abstehenden Fäden aus synthetischen Fasern separat, hergestellt wird und anschließend die abstehenden Fäden schräg zu ihrer Längserstreckung abgeschnitten werden.

## Claims

1. Wound cleansing assembly which comprises or is a wound cleansing cloth (1), which has at least one supporting layer (2) and threads (3) made from synthetic fibres arranged on the supporting layer (2) and protruding from the supporting layer (2), at least some of the threads (3) having freely protruding, preferably trimmed, ends (4) on the side thereof facing away from the supporting layer (2), the threads (3) having ends (4) or end surfaces which extend, preferably cut off, at an angle to the longitudinal extent (5) thereof, and the threads protruding from the supporting layer forming a pile arranged on the supporting layer and protruding from the latter, and the wound cleansing assembly being packed in a sterile manner in a packaging (7) which is preferably sealed in an air-tight manner, **characterised in that** the pile height is between 3 and 30 mm and the threads protruding from the supporting layer have a thickness of between 0.5 and 20 dtex.

2. Wound cleansing assembly according to claim 1, **characterised in that** the supporting layer (2), preferably the entire wound cleansing cloth (1), comprises synthetic fibres, preferably plastic fibres, is preferably made of synthetic fibres, preferably plastics fibres and/or that it is provided that that the synthetic fibres of the threads (3) and/or of the supporting layer (2), preferably of the entire wound cleansing cloth (1), are made of polyester and/or polyamide and/or acrylic polymer.

3. Wound cleansing assembly according to claim 1 or 2, **characterised in that** the threads (3) and/or the supporting layer (2) are/is coated with a coating compound, preferably made of 100% acrylic polymer.

4. Wound cleansing assembly according to one of the claims 1 to 3, **characterised in that** the supporting layer (2) is a meshed fabric, preferably a woven or knitted fabric, and preferably the threads (3) protruding from the supporting layer (2) are woven or knitted into the supporting layer (2).

5. Wound cleansing assembly according to claim 4, **characterised in that** the supporting layer (2) is coated, preferably on the side facing away from the protruding threads (3), with a, preferably continuous, reinforcing layer (6) connecting the stitches of the supporting layer (2).

6. Wound cleansing assembly according to one of the claims 1 to 5, **characterised in that** at least part of the threads (3) protruding from the supporting layer (2) and/or at least part of the threads (3) forming the supporting layer (2) contains or consists of, preferably pure, silver and/or, preferably pure, copper threads and/or synthetic threads with a silver coating and/or a copper coating.

7. Wound cleansing assembly according to one of the claims 1 to 6, **characterised in that** nanoparticles adhere to and/or are arranged on synthetic fibres of the supporting layer (2) and/or on the synthetic fibres of the threads (3) protruding from the supporting layer (2).

8. Wound cleansing assembly according to one of the claims 1 or 7, **characterised in that** the wound cleansing cloth (1) is, preferably interchangeably, arranged on and/or fastened to a, preferably rigid, supporting body (18) of the wound cleansing assembly.

9. Wound cleansing assembly according to one of the claims 1 to 8, **characterised in that** at least one reinforcing layer (6) is arranged in and/or on the supporting layer (2), preferably on the side of the supporting layer (2) facing away from the threads (3).

10. Wound cleansing assembly according to one of the claims 1 to 9, **characterised in that** synthetic fibres of the supporting layer (2) and/or the synthetic fibres of the threads (3) protruding from the supporting layer (2) are heat treated, preferably shrunk by means of heat treatment.

11. Use of synthetic fibres for manufacturing a wound cleansing assembly or a wound cleansing cloth (1) of a wound cleansing assembly according to one of the claims 1 to 10 for cleansing, preferably for debridement, of wounds or the skin.

12. Method for manufacturing a wound cleansing assembly according to one of the claims 1 to 10, **characterised in that** two supporting layers (2) are manufactured together in a first method step in the form of meshed fabric, preferably knitted or woven, wherein in said first method step, an intermediate layer (8) is formed of threads (3) extending between the two supporting layers (2) and worked into both supporting layers (2) between the two supporting layers (2) and, in a second method step, the threads (3) are parted, preferably cut through, preferably in the middle between the two supporting layers (2).

13. The method for manufacturing a wound cleansing assembly or a cloth according to one of the claims 1 to 10, **characterised in that** a wound cleansing cloth is manufactured separately, preferably by weaving or knitting, with the supporting layer and the threads made of synthetic fibres and protruding from the supporting layer, and subsequently the protruding threads are cut off at an angle to the longitudinal extent thereof.

## Revendications

1. Dispositif de nettoyage de plaie, qui présente ou qui est un tissu de nettoyage de plaie (1), lequel présente au moins une couche de support (2) et des fils (3) disposés sur la couche de support (2) et faisant saillie de ladite couche de support (2), ceux-ci étant des fils en fibres synthétiques, au moins certains des fils (3) présentant sur leur face opposée à la couche de support (2) des extrémités (4), de préférence découpées, dépassant librement, les fils (3) présentant des extrémités (4) ou surfaces d'extrémité, de préférence découpées, s'étendant de manière oblique par rapport à leur étendue longitudinale (5) et les fils faisant saillie de la couche de support formant un voile situé sur la couche de support en en faisant saillie, et le tissu de nettoyage de plaie étant emballé de manière stérile dans un emballage (7) fermé de préférence hermétiquement, **caractérisé en ce que** le voile présente une hauteur comprise entre 3 et 30 mm et **en ce que** les fils faisant saillie de la couche de support présentent entre 0,5 et 20 dtex.

2. Dispositif de nettoyage de plaie selon la revendication 1, **caractérisé en ce que** la couche de support (2), de préférence tout le tissu de nettoyage de plaie (1), présente des fibres synthétiques, de préférence des fibres plastiques, se compose de préférence de fibres synthétiques, de préférence de fibres plastiques, et/ou **en ce qu'**il est prévu que les fibres synthétiques des fils (3) et/ou de la couche de support (2), de préférence les fibres de tout le tissu de nettoyage de plaie (1), se composent de polyester et/ou de polyamide et/ou de polyacrylique.

3. Dispositif de nettoyage de plaie selon la revendication 1 ou 2, **caractérisé en ce que** les fils (3) et/ou la couche de support (2) sont enduits d'une matière de revêtement, de préférence constituée à 100 % de polyacrylique.

4. Tissu de nettoyage de plaie selon l'une quelconque des revendications 1 à 3, **caractérisé en ce que** la couche de support (2) est un article maillé, de préférence un tissu ou un article tricoté, et **en ce que** de préférence les fils (3) faisant saillie de la couche de support (2) sont intégrés lors du tissage ou incorporés lors du tricotage dans la couche de support (2).

5. Dispositif de nettoyage de plaie selon la revendication 4, **caractérisé en ce que** la couche de support (2) est enduite, de préférence sur la face opposée aux fils (3) faisant saillie, d'une couche de renforcement (6), de préférence traversante, reliant les mailles de la couche de support (2).

6. Dispositif de nettoyage de plaie selon l'une quelconque des revendications 1 à 5, **caractérisé en ce qu**'au moins une partie des fils (3) faisant saillie de la couche de support (2) et/ou au moins une partie des fils (3) formant la couche de support (2) sont de préférence des fils d'argent pur et/ou de préférence des fils de cuivre pur et/ou contiennent des fibres synthétiques pourvues d'un revêtement en argent et/ou d'un revêtement en cuivre ou en sont formés.

7. Dispositif de nettoyage de plaie selon l'une quelconque des revendications 1 à 6, **caractérisé en ce que** des nanoparticules adhèrent ou sont disposées au niveau des fibres synthétiques de la couche de support (2) et/ou au niveau des fibres synthétiques des fils (3) faisant saillie de la couche de support (2).

8. Dispositif de nettoyage de plaie selon l'une quelconque des revendications 1 à 7, **caractérisé en ce que** le tissu de nettoyage de plaie (1) est disposé et/ou est fixé au niveau d'un corps de support (18), de préférence rigide, du dispositif de nettoyage de plaie, de préférence de manière à pouvoir être changé.

9. Dispositif de nettoyage de plaie selon l'une quelconque des revendications 1 à 8, **caractérisé en ce qu**'au moins une couche de renforcement (6) est disposée dans et/ou au niveau de la couche de support (2), de préférence sur la face de la couche de support (2) opposée aux fils (3) faisant saillie.

10. Dispositif de nettoyage de plaie selon l'une quelconque des revendications 1 à 9, **caractérisé en ce que** des fibres synthétiques de la couche de support (2) et/ou les fibres synthétiques des fils (3) faisant saillie de la couche de support (2) sont traitées thermiquement, de préférence sont rétrécies au moyen d'un traitement thermique.

11. Utilisation de fibres synthétiques aux fins de la fabrication d'un dispositif de nettoyage de plaie ou d'un tissu de nettoyage de plaie (1) d'un dispositif de nettoyage de plaie selon l'une quelconque des revendications 1 à 10 aux fins du nettoyage des plaies ou de la peau, de préférence pour le débridement de ces dernières.

12. Procédé de fabrication d'un dispositif de nettoyage de plaie selon l'une quelconque des revendications 1 à 10, **caractérisé en ce que** deux couches de support (2) sont fabriquées, de préférence sont tricotées ou tissées, ensemble sous la forme d'un tissu maillé lors d'une première étape de procédé, lors de ladite première étape de procédé, une couche intermédiaire (8) constituée de fils (3) s'étendant entre les deux couches de support (2) et incorporés dans les deux couches de support (2) étant réalisée entre les deux couches de support (2) et les fils (3) étant séparés, de préférence découpés, lors d'une deuxième étape de procédé, de préférence au centre entre les deux couches de support (2).

13. Procédé de fabrication d'un dispositif de nettoyage de plaie ou d'un tissu selon l'une quelconque des revendications 1 à 10, **caractérisé en ce qu'**un tissu de nettoyage de plaie est fabriqué, de préférence par tissage ou par tricotage, de manière séparée avec la couche de support et avec les fils faisant saillie de la couche de support et constitués de fibres synthétiques, et **en ce qu'**immédiatement après les fils faisant saillie sont découpés de manière oblique par rapport à leur étendue longitudinale.
